# EUROPEAN PATENT APPLICATION

(11) **EP 1 659 170 A1**
(43) Date of publication of application: **24.05.2006**
(21) Application number: 04747408.5
(22) Date of filing: 12.07.2004
(51) Int. Cl.: C12M 1/00, C12Q 1/68, C12N 15/10

(54) **NUCLEIC ACID AMPLIFIER AND METHOD OF NUCLEIC ACID AMPLIFICATION**

(30) Priority: 11.07.2003 JP 2003273430
(71) Applicant: TAIYO YUDEN CO., LTD., Tokyo 110-0005 (JP)
(72) Inventor: HAGIWARA, Naoto, c/o TAIYO YUDEN CO., LTD., Tokyo 110-0005 (JP)
(74) Representative: Goddar, Heinz J.
(86) International application number: PCT/JP2004/009942
(87) International publication number: WO 2005/005594

(57) **Abstract**

A nucleic acid amplifier capable of efficiently performing PCR even with the use of nucleic acid synthetase lacking heat resistance, which nucleic acid amplifier can realize nucleic acid synthetase reutilization, scaleup, etc. and facilitates separation/purification of nucleic acid after amplification; and a method of nucleic acid amplification performed therewith. In particular, the method comprises causing at least a nucleic acid as a template, a nucleic acid as a primer and a reaction solution containing a phosphate compound and a metal ion to flow through a channel having a region for melting an intramolecularly and/or intermolecularly formed double strand of nucleic acid so as to effect denaturation into single strands and a regeneration region for regenerating a double strand of nucleic acid from the nucleic acid after the double strand melting, thereby melting at the denaturation region the intramolecularly and/or intermolecularly formed double strand of nucleic acid as the template so as to effect denaturation into single strands and thereafter at the regeneration region not only regenerating a double strand between the template nucleic acid after the double strand melting and the primer nucleic acid but also carrying out nucleic acid synthesis with the nucleic acid synthetase fixed within the regeneration region.

## Description

### Technical Field

The present invention relates to a nucleic acid amplifier and a method of nucleic acid amplification which utilizes a PCR method. More specifically, the present invention relates to a nucleic acid amplifier having at least one flow channel therein, in which a reaction solution containing at least a nucleic acid to be used as a template, a nucleic acid to be used as a primer, a phosphate compound, and a metal ion is introduced into the flow channel to thereby carry out the nucleic acid amplification by means of a nucleic acid synthetase immobilized on the flow channel, and to a method of nucleic acid amplification performed therewith.

### Background Art

For efficient replication and amplification of a minute amount of template DNA, a polymerase chain reaction (PCR) method has been widely used. The PCR method is a method of amplifying a target DNA involving one cycle of the steps of: forming single-stranded DNAs by thermal denaturation of a double-stranded DNA provided as a template; annealing each of the obtained single-stranded DNAs with its complementary primer; and synthesizing a double-stranded DNA by forming a complementary strand from the primer by the action of a heat-resisting DNA polymerase, the cycle being repeated two or more times.

Each of the above steps is carried out with managements on the temperature of a reaction solution and reaction time. Generally, the thermal denaturation of a double-stranded DNA provided as a template to single-stranded DNAs is carried out at about 94 °C, the annealing of a primer to each of the single-stranded DNAs is carried out at about 55°C, and the synthesis of a complementary strand with a DNA polymerase is carried out at about 72°C.

Conventionally, a device which has been known in the art as a device that performs a PCR method automatically is of placing a reaction solution containing template DNA, primers, dNTPs, DNA polymerase, and the like in an Eppendorf tube and then inserting the tubes in the respective wells formed in an aluminum block to carry out reactions by changing the temperature of the block using a heater and a cooler.

However, the PCR method requires heat cycles be carried out under accurate temperature-controls. Any reaction in a batch system like the one described above has been limited in scale-up because thermal fluctuation in a reaction system increases extensively as the scale of the reaction increases.

Therefore, as a PCR method capable of carrying out the temperature-control of the heat cycles with high accuracy and also permitting scale-up, a flow type PCR method is disclosed in Patent Document 1 and Non-Patent Document 1 listed below. This flow type PCR method is a method involving carrying out heat cycles by introducing a reaction solution containing DNA polymerase, template DNA, primer DNAs, dNTPs, and so on into a flow channel having a heating portion and a cooling portion.

In addition, in Patent Document 2 listed below, there is disclosed a method of nucleic acid sequence amplification, characterized by including the steps of: (a) synthesizing a primer-elongation chain complimentary to a template by treating a nucleic acid to be used as the template with at least one primer substantially complimentary to the base sequence of the nucleic acid and a DNA polymerase, in which the primer is a chimera oligonucleotide primer containing deoxyribonucleotides and ribonucleotides, the ribonucleotides being arranged on the 3' end or 3' direction thereof for being cleaved by an endonuclease; (b) cleaving a ribonucleotide-containing moiety of the primer-elongation chain of a double-stranded nucleic acid obtained in the step (a) by means of endonuclease; and (c) carrying out chain substitution by elongating a nucleic acid sequence complementary to the template by means of a DNA polymerase having chain-substitution activity from the 3' end of the primer portion of the double-stranded nucleic acid obtained in the step (b), from which the primer-elongation chain is cleaved. According to this method (ICAN method), DNA can be amplified without any heat cycle, so that an enzyme having no heat resistance property can be used and a reaction scale is not restricted by thermal fluctuation.
Patent Document 1: JP 06-30776 A
Patent Document 2: JP 2003-70490 A
Non-Patent Document 1: "Science" (1998) 280 5366, p.1046-1048 (Written by Kopp MU, Mello AJ, and Manz A.)

### Disclosure of the Invention

### Problems to be solved by the Invention

However, any of the above batch type PCR method and the PCR methods described in Patent Document 1 and Non-Patent Document 1 requires heating in denaturation of a double-stranded template DNA to single-stranded DNAs, and therefore a specific DNA polymerase having heat resistance property is required. Therefore, there is a disadvantage in that none of the DNA polymerases having no heat resistance property, which generally exist in nature, can be used.

In addition, the method disclosed in the above Patent Document 2 employs a chimera primer composed of RNA and DNA as a primer, or requires a specific enzyme such as exo-Bca DNA polymerase that synthesizes DNA while winding off the double strand of DNA and RNase H which cleaves the contact point between DNA additionally elongated from the chimera primer and a chimera primer RNA. Thus, there is a disadvantage of increasing cost.

Furthermore, in the above conventional methods, the reaction product is contaminated with nucleic acid synthetases such as DNA polymerase. Thus, the purification of amplified DNA will take much time and almost no recycle of expensive nucleic acid synthetases was possible.

Therefore, an object of the present invention is to provide a nucleic acid amplifier by which PCR can be continuously performed in an efficient manner not only in the case of using a nucleic acid synthetase having heat resistance property but also in the case of using one having no heat resistance property, the nucleic acid synthetase can be recycled and continuously utilized, and also the reaction can be scaled up while the isolation and purification of an amplified nucleic acid are facilitated, and a method of nucleic acid amplification performed therewith.

### Means for solving the Problems

In order to achieve the objects, the nucleic acid amplifier of the present invention is a nucleic acid amplifier having at least one flow channel therein, in which a reaction solution containing at least a nucleic acid to be used as a template, a nucleic acid to be used as a primer, a phosphate compound, and a metal ion is caused to flow through the flow channel to thereby perform the nucleic acid amplification in the flow channel, characterized in that the flow channel includes: a denaturation region in which a denaturation reaction is carried out, the denaturation reaction including melting an intramolecularly and/or intermolecularly formed double strand of the nucleic acid to be used as the template; a regeneration region in which a double strand is formed with the nucleic acid to be used as the template after the double strand thereof is melted and the nucleic acid to be used as the primer; and a nucleic acid synthetase immobilized in the regeneration region.

According to the nucleic acid amplifier of the present invention, when a reaction solution containing at least a nucleic acid to be used as a template, a nucleic acid to be used as a primer, a phosphate compound, and a metal ion is introduced into at least one flow channel having both the denaturation region and the regeneration region to thereby synthesize a nucleic acid, the nucleic acid synthetase immobilized on the regeneration region is not influenced by heating or the like in denaturing by melting the nucleic acid to be used as a template into single strands. Thus, the nucleic acid synthetase is prevented from deactivation, so that PCR can be carried out continuously even if any nucleic acid synthetase having no heat resistance property is used. In addition, as the nucleic acid synthetase is being immobilized, the isolation and purification of an amplified nucleic acid can be easily carried out. Besides, the nucleic acid synthetase can be recycled and continuously utilized, and the scale-up of the reaction can be also facilitated. Here, in the present invention, the term "nucleic acid" means any of nucleic acids that include those of both natural and non-natural types.

The nucleic acid amplifier of the present invention preferably includes a means for controlling temperature which is capable of heating the denaturation region and of keeping a temperature of the regeneration region lower than a temperature of the denaturation region. According to this aspect of the present invention, a series of PCR cycles can be continuously carried out in an efficient manner, in which each cycle includes the steps of: thermally melting a nucleic acid to be used as a template, which includes a double strand formed in a molecule and/or between molecules to denature the nucleic acid into single strands; forming double strands between the nucleic acids each to be used as the template resulted from the molten double strand and complementary primers thereto under an environment having a temperature lower than that of the denaturation region; and synthesizing complementary strands from the primers by reacting with a nucleic acid synthetase under an environment having a temperature lower than that of the denaturation region.

The nucleic acid synthetase is preferably immobilized on beads, the beads filling at least the regeneration region. According to this aspect of the present invention, the immobilized nucleic acid synthetase can be efficiently contacted with a reaction solution, to thereby increase the reaction efficiency.

The nucleic acid synthetase may be immobilized at least on the inner wall surface of the regeneration region. According to this aspect of the present invention, the flow channel on which the nucleic acid synthetase is immobilized can be easily formed. In other words, for the formation of such a flow channel, the flow channel may be formed such that the nucleic acid synthetase is immobilized on the whole surface of the flow channel at first. Such an aspect allows an enzyme in the regeneration region to be retained in an active state even though an enzyme in the denaturation region is deactivated. Therefore, a desired flow channel can be easily formed.

Furthermore, the denaturation region and the regeneration region are provided alternately in the flow channel. According to this aspect of the present invention, two or more PCR cycles are carried out and thus the target nucleic acid can be efficiently amplified.

In the nucleic acid amplifier of the present invention, a nucleic acid synthetase having an optimum temperature of 30 to 40°C can be used as the nucleic acid synthetase. According to the aspect of the present invention, nucleic acid synthetases for intended usages can be selected from an extended range, so that any of comparatively cost-effective enzymes which could not be used in the conventional PCR can be chosen. In addition, it becomes possible to concomitantly use any general enzyme other than nucleic acid synthetases. Thus, for example, an enzyme which has been hardly used together in the conventional PCR, such as one that corrects a mismatch in synthesized nucleic acid, can be also used to improve the reliability of amplification compared with that of the conventional PCR.

In the nucleic acid amplifier of the present invention, the flow channel may provide a circulation flow channel, and the circulation flow channel may include the regeneration region and the denaturation region.

Here, the term "circulation flow channel" refers to a flow channel for circulating a reaction solution and allowing the reaction solution to alternatively pass through the denaturation region and the regeneration region in the circulation flow channel, such as a flow channel having a loop structure in which the flow channel is branched at a predetermined place and then the branched channels are joined again to each other, and a flow channel composed of one or more flow channels in the form of a loop structure as a whole of the flow channel in which the circulation of part or whole of flow can be performed by passing through the loop structure of flow channels.

According to this aspect of the present invention, the nucleic acid to be used as a template, the nucleic acid to be used as a primer, the phosphate compound, or the like can be repetitively fed to the denaturation or regeneration region while being circulated in a predetermined region. Therefore, the template can be prevented from depletion and the reaction solution can be recycled positively, so that running costs can be reduced.

Further, the nucleic acid amplifier of the present invention preferably includes a solution-sending device for directionally regulating a flow of the reaction solution, and the solution-sending device is preferably controllable to periodically reverse the direction of flow of the reaction solution. According to this aspect of the present invention, various kinds of solution-sending devices each of which can send a solution within a limited capacity in volume can be used. Thus, the solution-sending device can be easily simplified, thereby favorably coping with the miniaturization of the device.

The method of amplifying a nucleic acid of the present invention is a method of amplifying a nucleic acid, the nucleic acid being used as a template in a reaction solution containing at least the nucleic acid to be used as the template, a nucleic acid to be used as a primer, a phosphate compound, and a metal ion, including the steps of: (a) denaturing the nucleic acid to be used as the template by melting an intramolecularly and/or intermolecularly formed double strand thereof at a predetermined region; (b) regenerating a double strand by forming the double strand between the nucleic acid obtained in step (a) that to be used as the template wherein the double strand is melted and the nucleic acid to be used as the primer at a region different from the region of the step (a); and (c) contacting the reaction solution during and/or just after the step (b) with a nucleic acid synthetase immobilized and retained in an active state at a region including the region on which the step (b) is performed.

According to the method of nucleic acid amplification of the present invention, it is possible to carry out the denaturation step and the regeneration step in a differentiated region for each. The nucleic acid synthetase immobilized on the region including a region where the regeneration step is conducted is not influenced by heating or the like in denaturing the nucleic acid to be used as a template. Thus, the nucleic acid synthetase can be prevented from deactivation, so that PCR can be carried out continuously even if any nucleic acid synthetase having no heat resistance property is used. In addition, as the nucleic acid synthetase is being immobilized, the isolation and purification of an amplified nucleic acid can be easily carried out. Besides, the nucleic acid synthetase can be recycled and continuously utilized, and the scale-up of the reaction can be also facilitated.

### Effects of the Invention

According to the present invention, when a nucleic acid synthesis reaction is carried by introducing a reaction solution containing at least a nucleic acid to be used as a template, a nucleic acid to be used as a primer, a phosphate compound, and a metal ion into a flow channel having: a region where an intramolecularly and/or intermolecularly formed double strand of the nucleic acid is melted and denatured into single-stranded nucleic acids; and a regeneration region where a double strand is reformed with the nucleic acids obtained by melting the double strand, a nucleic acid synthetase immobilized on the regeneration region is not influenced by heating or the like in denaturation of a nucleic acid to be used as a template. Thus, the nucleic acid synthetase can be prevented from deactivation, so that PCR can be carried out continuously even if any nucleic acid synthetase having no heat resistance property is used. In addition, as the nucleic acid synthetase is being immobilized, the isolation and purification of an amplified nucleic acid can be easily carried out. Besides, the nucleic acid synthetase can be recycled and continuously utilized. Therefore, the scale-up of the reaction can be also facilitated.

### Brief Description of the Drawings

[Fig. 1] A diagram that illustrates an embodiment of the nucleic acid amplifier of the present invention.
[Fig. 2] A schematic diagram of part of a flow channel of the nucleic acid amplifier shown in Fig. 1.
[Fig. 3] A diagram that illustrates another embodiment of the nucleic acid amplifier of the present invention.
[Fig. 4] A schematic diagram of a circulation flow channel of the nucleic acid amplifier.
[Fig. 5] An explanatory diagram of a temperature-control means for denaturation and a temperature-control means for regeneration for forming a denaturation region and a regeneration region, respectively.
[Fig. 6] A diagram that illustrates still another embodiment of the nucleic acid amplifier of the present invention.
[Fig. 7] A schematic diagram that illustrates a nucleic acid synthetase immobilized in a capillary in the nucleic acid amplifier. [Fig. 8] A schematic diagram that illustrates a single unit of flow channels in the nucleic acid amplifier used in Example of the present invention.
[Fig. 9] A photograph showing the results of detection with agarose gel electrophoresis after nucleic acid amplification with the nucleic acid amplifier.

### Description of Symbols

1, 1a to 1g base plates
2 flow channel
2a injection pore
2b discharge pore
2c branched flow channel
3 beads-filling part
3a enlarged diameter portion
4 beads
5 nucleic acid synthetase
6 immobilized nucleic-acid synthesizing enzyme
10, 20, 50 nucleic acid amplifier
11 to 13, 13a external solution-sending device
14 first reaction solution chamber
15 second reaction solution chamber
16 reaction solution chamber
31, 32 thermostatic chamber
33, 52 temperature-controlling device
34, 39 temperature-control means for denaturation
35, 40 temperature-control means for regeneration
36, 37 stirrer
38 partition plate
51 capillary
A denaturation-temperature region
B regeneration-temperature region

### Best Mode for carrying out the Invention

At first, the method of nucleic acid amplification of the present invention will be described.

The method of nucleic acid amplification of the present invention involves introducing a reaction solution containing at least a nucleic acid to be used as a template (hereinafter, simply referred to as a template), a nucleic acid to be used as a primer (hereinafter, simply referred to as a primer), a phosphate compound, and a metal ion into at least one flow channel to denature the template in the flow channel, executing annealing between the denatured template and the primer, and synthesize a nucleic acid with a nucleic acid synthetase. The flow channel is constructed of: a denaturation region for carrying out a denaturation reaction of the double-stranded nucleic acid to be used as a template; and a regeneration region for carrying out an annealing reaction between the single-stranded nucleic acid to be used as a template and the primer and also carrying out a nucleic acid synthesis reaction with a nucleic acid synthetase, where the nucleic acid synthetase is immobilized on at least part of the regeneration of the flow channel. Here, the term "denaturation of a template" means that a double-stranded nucleic acid is melted and converted to single-stranded nucleic acids.

The denaturation region is set to an environment required for the denaturation of a template, for instance, set to any of environmental conditions of (1) being adjusted to the melting temperature of the nucleic acid or higher, (2) being adjusted to acidic or basic, (3) containing no cation, or (4) being mixed with a hydrogen-bond inhibitor (e.g., urea or guanidium salt).

In the present invention, as the denaturation and regeneration of a nucleic acid are repetitively carried out, among the above conditions, it is preferable to set to one being adjusted to the melting temperature of the nucleic acid or higher (heating is more effective as means) or being adjusted to acidic or basic because it can be set repetitively. Particularly preferable is to adjust to the melting temperature of the nucleic acid or higher because it is most effective. For instance, the template is denaturated by heating at a temperature equal to the melting temperature of the nucleic acid or higher, the template may be heated at 90 to 99°C, preferably 92 to 97°C, when the template has a length of several hundred mer although the temperature varies from case by case depending on the length or arrangement of the template.

Here, it is difficult to impart resistance to bases to a nucleic acid synthetase. In the conventional PCRmethod, therefore, no basic environment has been used as a template denaturation condition. In the present invention, however, the region on which the nucleic acid synthetase is immobilized may be set to a neutral environment. Therefore, as far as being set to the neutral environment, the denaturation region can be set to a basic environment so that the template denaturation is carried out.

On the other hand, the regeneration region is set to an environment required for the nucleic acid regeneration, for example, any of the environment that satisfies all of conditions of (1) being adjusted to the melting temperature of a nucleic acid or lower (by means of non-heating or cooling), (2) being adjusted to be a mild acid or mild base (approximately pH 7 ± 3), (3) containing appropriate cations, and (4) containing no hydrogen-bond inhibitor (e.g., urea or guanidium salt). For instance, the temperature for carrying out the nucleic acid regeneration, which varies from case by case depending on the melting temperature depending on the template and the primer, may be, for example, 30 to 70°C when the primer of 15 to 30 mer is used. In the present invention, the temperature is particularly preferably 30 to 40°C. Here, the term "nucleic acid regeneration" means the formation of a double strand between single-stranded nucleic acids complementary to each other. Thus, the nucleic acid regeneration under the environment for carrying out PCR substantially means annealing between the template and the primer.

In the present invention, the movement of the reaction solution introduced into the flow channel toward the denaturation region allows the reaction solution to be exposed under the environmental conditions defined for the denaturation region. In addition, the movement to the regeneration region allows the reaction solution to be exposed under the environmental conditions defined for the regeneration region.

Furthermore, in the present invention, for heating the reaction solution moving in the flow channel to the melting temperature of the nucleic acid or higher, the denaturation region is preferably formed by means of a temperature-control means for denaturation mounted outside the flow channel. On the other hand, for adjusting the reaction solution moving in the flow channel to the melting temperature of the nucleic acid or lower, the regeneration region is preferably formed by means of a temperature-control means for regeneration mounted outside the flow channel.

The nucleic acid synthetase used in the method of the present invention is an enzyme which can be used for the nucleic acid amplification, and is not specifically limited as far as it is any of those which are commonly available. Concrete examples of the enzymes include DNA polymerase, ligase, reverse transcriptase, and RNA polymerase. In addition, the nucleic acid synthetase may be any combination thereof.

Here, in the present invention, a nucleic acid synthetase having heat resistance property, which has been used in the conventional PCR or ligase chain reaction (LCR) method, may be used. As the nucleic acid synthetase is immobilized on the flow channel of the regeneration region without being exposed to heat or the like generated in the template denaturation, any nucleic acid synthetase having no heat resistance property can be used.

In the present invention, one having an optimum temperature of 30 to 40°C can be suitably used as a nucleic acid synthetase. Thus, any of comparatively cost-effective enzymes which could not be used in the conventional PCR can be chosen. In addition, it becomes possible to use any of general enzymes concomitantly except other nucleic acid synthetases. Thus, an enzyme which has been hardly used together in the conventional PCR, such as one that corrects a mismatch in synthesized nucleic acid, can be also used to improve the reliability of amplification compared with that of the conventional PCR.

In the present invention, an enzyme having high reaction efficiency or an enzyme easily obtainable can be preferably used. Concretely, DNA polymerase I derived from *Escherichia coli*, which showshigh fidelityin replication,ispreferably used. Inaddition, a Klenow fragment or the like prepared by removing an exonuclease active site from the DNA polymerase I may be used though fidelity in replication slightly reduces.

The nucleic acid synthetase may be, for example, immobilized on the surface of beads and then filled in at least part of the regeneration region of the flow channel, or may be directly immobilized on the inner wall surface of the flow channel.

Here, when nucleic acid synthetase immobilized on the beads is used, the immobilized nucleic-acid synthesizing enzyme can be efficiently brought into contact with the reaction solution, so that the reaction efficiency can be raised.

When the nucleic acid synthetase is immobilized at least on the inner wall surface of the regeneration region, the device of the present invention can be configured simply. In other words, when such a kind of flow channel is formed, at first, the entire flow channel can be formed by immobilizing the nucleic acid synthetase on the entire surface of the flow channel. This embodiment allows a desired flow channel to be easily formed because the enzyme in the regeneration region retains its active state even if the enzyme in the denaturation region is deactivated.

Examples of the material of beads for immobilizing the nucleic acid synthetase may preferably include, but not specifically limited to, metal fine particles, glass particles, and resin particles. In particular, beads having good affinity to a biomolecule and capable of immobilizing an enzyme thereon easily, such as latex beads and chitosan beads, are preferably used. The size of each of the beads may be any size enough to be filled in the flow channel and may be suitably defined, but is generally 0.4 to 100 µm, preferably 1 to 50 µm in diameter.

In addition, the flow channel may be preferably formed of a material having comparatively high heat conductivity, stability in the temperature range required for PCR, resistance to erosion with an electrolytic solution or an organic solvent, and difficulty in adsorption of nucleic acid or protein. Examples of materials having heat resistance property and corrosion resistance include glass, quartz, silicon, and various kinds of plastics. Furthermore, it is preferable that the surface of any of those materials (the inner wall surface to be in contact with the reaction solution) be coated with a material, such as polyethylene and polypropylene, generally known to be difficult in adsorption of nucleic acid or protein. Alternatively, it is preferable to prevent the adsorption of nucleic acid or protein by introduction of any molecule rich in hydrophilic functional groups, such as polyethylene glycol (PEG), via a covalent bond or the like.

Any of well-known methods including a supporting or inclusion method, a covalent-binding method, a cross-linking method, and an electrostatic adsorption method may be adopted as a method of immobilizing the nucleic acid synthetase on the surface of the beads or the inner wall surface of the flow channel. For repeating the enzyme reaction, among them, particularly preferable is the covalent-binding method or cross-linking method. For instance, the covalent-binding method can be performed on the basis of the method described in JP-A-3-164177. A comparatively highly reactive functional group (e.g., a chlorocarbonyl group (carboxylate chloride), a carboxyl group, an amino group, a thiol group (sulfanyl group), or an epoxy group) may be introduced into the surface of beads or the inner wall surface of the flow channel to allow such a functional group to react with a carbonyl group, an amino group, or a thiol group (sulfanyl group) on the surface of the nucleic acid synthetase, thereby attaining the immobilization.

The reaction solution used in the present invention may contain at least a template, a primer, a phosphate compound, and a metal ion.

The template described above is a nucleic acid, an amplification target, which may be any of natural or non-natural type nucleic acids prepared by the conventional method. The concentration of the template in the reaction solution is, in general, preferably 0.01 to 100 pM, more preferably 0.1 to 10 pM.

The primer is a nucleic acid having a base sequence complementary to at least part of the base sequence of the template and may be any of those used in the common PCR or LCR method. However, it is preferable to design such a primer so as to efficiently amplify the target nucleic acid, and one having a length of 15 to 30 mer is generally preferably used. For instance, the nucleic acid to be used as a primer may be one easily prepared using an automated polynucleotide synthesizer. The concentration of the primer in the reaction solution is, in general, preferably 0.01 to 1 µM, more preferably 0.1 to 0.2 µM.

Furthermore, the primers described above include chemically modified or altered non-natural type nucleic acids for a subsequent detection or isolation process. Preferable examples of the above non-natural type nucleic acids include, but not specifically limited to, oligonucleic acids labeled with biotin or FITC, oligonucleic acids having phosphotioate bindings, and chimeric nucleic acid containing peptide nucleic acid (PNA) and natural type nucleic acid.

The phosphate compound is a component to be provided as a substrate for the amplification of nucleic acid. For instance, when a DNA polymerase or a reverse transcriptase is used as a nucleic acid synthetase, a mixture containing dNTPs (i.e., dATPs, dCTPs, dGTPs, and dTTPs) at any ratio, preferably four kinds of deoxynucleotide triphosphate at equal ratio may be used. On the other hand, when ligase is used, it is preferable to use NTP, and ATP or GTP can be particularly preferably exemplified. The concentration of the phosphate compound in the reaction solution can be suitably defined. In general, however, it is preferably 0.01 to 1 mM, more preferably 0.1 to 0.5 mM.

For the metal ion, a potassium ion (K⁺), a sodium ion (Na⁺), or a magnesium ion (Mg²⁺) may be exemplified. Including such a metal ion makes it possible to attain effects on improvements in stability of double-stranded nucleic acid, enzyme activity, and faithfulness of synthesized nucleic acid. In general, the concentration of the metal ion in the reaction solution is preferably 10 to 200 mM, more preferably 50 to 100 mM for the potassium or sodium ion. Alternatively, for the magnesium ion, the concentration is preferably 1 to 5 mM, more preferably 1.5 to 2.5 mM.

In the method of the present invention, for effectively carrying out the denaturation of a template, the annealing between the denatured template and the primer, and the synthesis of nucleic acid in the flow channel, it is preferable that condition such as the rate of sending the reaction solution and the length of flow channel be suitably adjusted. Those conditions may vary from case by case depending on the length of the template, the length of the nucleic acid to be synthesized, the reaction rate with the nucleic acid synthetase used, or the like. In general, however, the time period required for the reaction solution to pass through the denaturation region once is 1 to 60 seconds, preferably 5 to 30 seconds, and also the time period required for the solution to pass through the regeneration region once is 5 to 300 seconds, preferably 10 to 120 seconds.

Hereinafter, the nucleic acid amplifier used in the method of nucleic acid amplification of the present invention will be described with reference to the attached drawings, but basically the same parts are provided with the same reference numerals or signs to omit the explanations thereof.

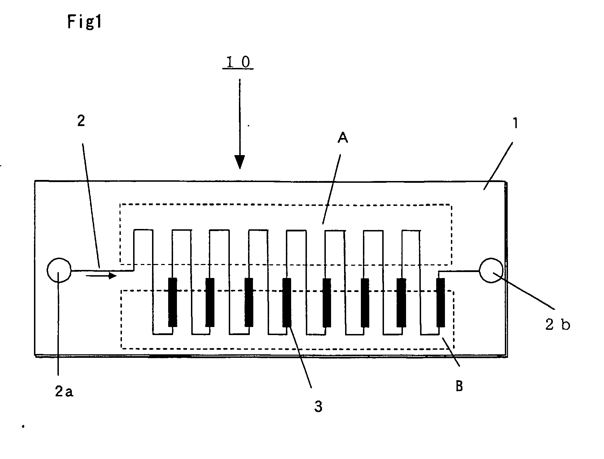

Fig. 1 illustrates one of the embodiments of the nucleic acid amplifier of the present invention. A nucleic acid amplifier 10 includes: a base plate 1 having a denaturation-temperature region A and a regeneration-temperature region B; and a flow channel 2 formed on the base plate, the flow channel 2 having a predetermined inner diameter and passing through both the denaturation-temperature region A and the regeneration-temperature region B two or more times while snaking its way alternately in the denaturation-temperature region A and the regeneration-temperature region B. Consequently, the flow channel 2 can be provided as one having a denaturation region for carrying out a denaturation reaction by which the nucleic acid to be provided as a template is converted to single strands and a regeneration region for further carrying out a nucleic acid synthesis reaction after annealing between the single-stranded nucleic acid and the primer. Part of the regeneration region of the flow channel is provided with plural beads-filling parts 3 in which beads having the nucleic acid synthetase immobilized on the surface thereof. In addition, both sides of the flow channel 2 are provided with an injection pore 2a for injecting the reaction solution into the flow channel and a discharge pore 2b for discharging the reaction solution after completion of the amplification reaction of nucleic acid.

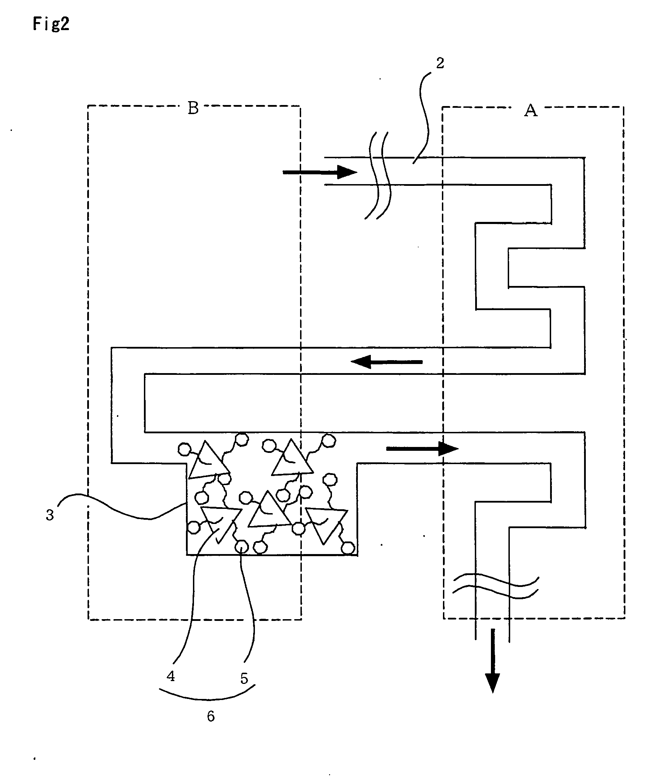

Furthermore, Fig. 2 is an enlarged schematic diagram of a part of the flow channel of the nucleic acid amplifier. The beads-filling part 3 is filled with an immobilized nucleic-acid synthesizing enzyme 6, which is prepared by immobilizing a nucleic acid synthetase 5 on the surface of beads 4, such that a reaction solution which has moved along the flow channel can contact with the nucleic acid synthetase 5 immobilized on the immobilized nucleic-acid synthesizing enzyme 6. Furthermore, when the immobilized nucleic-acid synthesizing enzyme 6 is filled in the flow channel, for preventing the leakage of the immobilized nucleic-acid synthesizing enzyme 6, it is preferable to install a filter having an appropriate filtration size on each of the inlet and outlet of the beads-filling part 3. Examples of a material of the filter preferably include, but not specifically limited to, one on which any nucleic acid is hardly absorbed, such as cellulose.

When the nucleic acid amplifier 10 is used, an external solution-sending device (not shown) such as a pump is employed to feed a reaction solution containing at least a template, a primer, a phosphate compound, and a metal ion in the direction along the arrow shown in the figure. Consequently, after the template has been converted into single strands in the denaturation region of the flow channel 2 by means of thermal denaturation, the single-stranded template is subjected to an annealing reaction with a primer complementary to the template in the regeneration region of the flow channel. Furthermore, a complementary strand with respect to the single-stranded template is synthesized by means of the immobilized nucleic-acid synthesizing enzyme 6 in the beads-filling part 3. Therefore, one cycle of PCR is carried out every time the reaction solution passes through both of the denaturation and regeneration regions of the flow channel.

In the present invention, the flow channel 2 may be formed such that the flow channel passes each of the denaturation-temperature region and the regeneration-temperature region of the base plate once or more. For efficiently carrying out the nucleic acid amplification, it is preferable to make the flow channel so as to pass through each of them 20 to 40 times.

In addition, the size of the flow channel 2 is preferably defined such that thermal fluctuation can be prevented through facilitating heat conduction by extending a specific surface area while reducing the diameter of the flow channel 2 (see Science No. 280, vol. 5366, pages 1046-1048 (written by Kopp MU, Mello AJ, and Manz A) , 1998). In the present invention, the optimal width of the flow channel is 20 to 200 µm, preferably 50 to 100 µm, and the optimal depth thereof is 20 to 200 µm, preferably 40 to 100 µm. Furthermore, the width of the flow channel corresponding to the portion to be filled with the immobilized nucleic-acid synthesizing enzyme 6 is 20 to 3,000 µm, preferably 50 to 1,000 µm, and the depth thereof is 20 to 1,000 µm, preferably 40 to 500 µm.

In addition, the flow channel 2 may be preferably formed of a material having comparatively high heat conductivity, stability in the temperature range required for PCR, resistance to erosion with an electrolytic solution or an organic solvent, and difficulty in adsorption of nucleic acid or protein. Examples of materials having heat resistance property and corrosion resistance include glass, quartz, silicon, and various kinds of plastics. Furthermore, it is preferable that the surface of any of those materials (the surface to be in contact with the reaction solution) be coated with a material, such as polyethylene and polypropylene, generally known to be difficult in adsorption of nucleic acid or protein. Alternatively, it is preferable to prevent the adsorption of nucleic acid or protein by introduction of any molecule rich in hydrophilic functional groups, such as polyethylene glycol (PEG), via a covalent bond or the like.

The base plate having the flow channel can be, for example, formed as follows. That is, a process may be suitably adopted, which involves: forming, on a single base plate made of the above material, a groove having the predetermined width and depth as defined above by cutting work or the like; and attaching another base plate or a film so as to cover the groove.

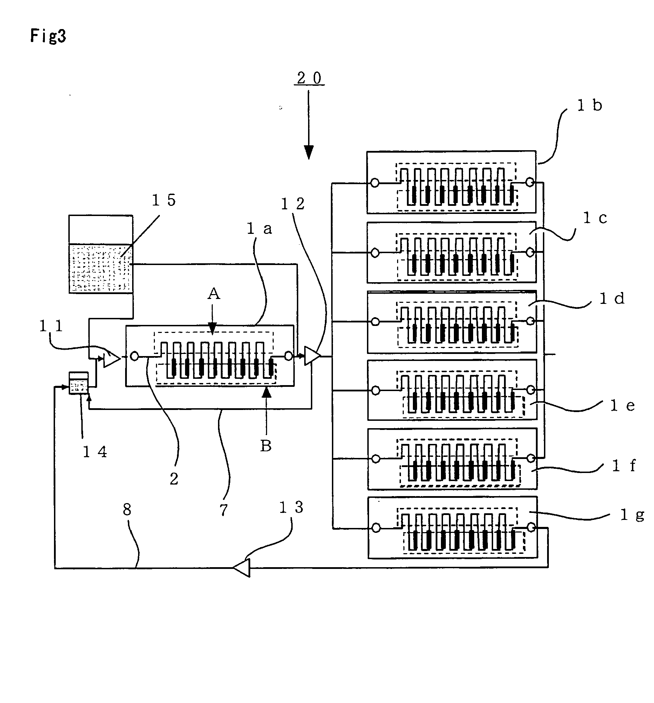

Fig. 3 illustrates another embodiment of the nucleic acid amplifier of the present invention. This nucleic acid amplifier 20 is designed such that the base plate 1a shown in Fig. 1 is connected to a plurality of other base plates 1b, 1c, 1d, 1e, 1f, and 1g in a branched configuration. The connections of those base plates are not limited to the configuration shown in Fig. 3. Any of various configurations may be chosen for performing efficient nucleic acid amplification.

The nucleic acid amplifier 20 employs a reaction solution consisting of a first reaction solution containing at least the above template and a second reaction solution containing at least the above primer, the phosphate compound, and the metal ion. At first, by means of an external solution-sending device 11 such as a pump, the first reaction solution and the second reaction solution are supplied to the base plate 1a from the first reaction solution chamber 14 and the second reaction solution chamber 15, respectively. Then, the reaction solution having passed through the base plate 1a is supplied directly by means of the external solution-sending device 12 as being a template to the base plates 1b, 1c, 1d, 1e, 1f, and 1g. For refilling reaction substrates such as the primer and the phosphate compound which have been consumed in the reaction at the base plate 1a, it is also configured that the second reaction solution can be supplied to the base plates 1b, 1c, 1d, 1e, 1f, and 1g from the second reaction solution chamber 15.

Then, the reaction solution having passed through the base plates 1b, 1c, 1d, 1e, and 1f may be directly recovered and the nucleic acid may be then purified. Alternatively, a plurality of additional base plates may be connected if required to carry out the amplification of nucleic acid.

Here, in this embodiment, flow channels 7 and 8 and a pump 13 are provided, thereby recycling part of the reaction solution having passed through the base plate 1a and one having passed through the base plate 1g, the reaction solution being recycled as the first reaction solution. By making such a recycling flow channel, the template can be prevented from depletion, so that the continuous amplification of nucleic acid can be stably carried out, thereby allowing reductions in running costs.

Furthermore, when the base plates are connected to each other in a branched configuration, it is preferable that a nucleic acid synthetase having high fidelity of replication be immobilized on at least one base plate on each stage, for example, a base plate (base plate 1a) just before branching and a base plate (base plate 1g) having a channel connected for recycling a reaction solution having passed through the base plate as a first reaction solution. Consequently, the template amplification can be performed precisely, so that the template can be precisely amplified even if PCR is carried out repetitively.

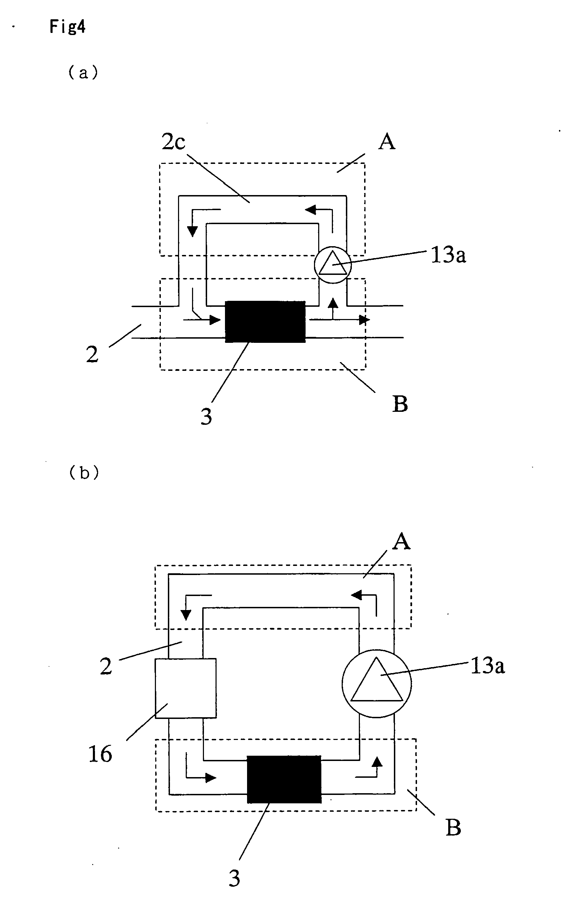

Figs. 4 (a) and (b) illustrate the configuration of a circulation flow channel, in the nucleic acid amplifier of the present invention, in which a reaction solution is circulated and is then alternately passed through the denaturation region and the regeneration region of the circulation flow channel.

In the circulation flow channel shown in Fig. 4 (a), the branched flow channel 2c branched at a predetermined site of the flow channel 2 forms a circulation flow channel and the sending of solution to the branched flow channel 2c is then controlled by the external solution-sending device 13a that directionally regulates the flow of the reaction solution. The reaction solution introduced into the branched flow channel 2c at the branched portion of the flow channel passes the denaturation region in the circulation flow channel through the denaturation-temperature region A and returns to the regeneration-temperature region B from a confluence portion of the flow channel, so that the reaction solution can be passed again through the regeneration region of the circulation flow channel through which the reaction solution has passed.

Furthermore, the above circulation flow channel may be configured such that, as shown in Fig. 4(b), the flow channel 2 may be formed in a loop shape so that no branched flow channel be provided. Here, the reaction solution chamber 16, provided as an inlet or outlet portion of the reaction solution, is placed on the middle of the loop-shaped flow channel2. Thus, the reaction solution introduced from the reaction solution chamber 16 circulates in the flow channel 2 in the direction of the arrow in the figure by means of an external solution-sending device 13a.

The reaction solution circulates through the circulation flow channel and then passes repetitively through the denaturation region and the regeneration region in the circulation flow channel in an alternate manner, thereby allowing a nucleic acid amplification reaction to proceed. The resulting amplification product can be collected from the outlet of the flow channel as shown in Fig. 4 (a) as well as from the reaction solution chamber 16 as shown in Fig. 4(b).

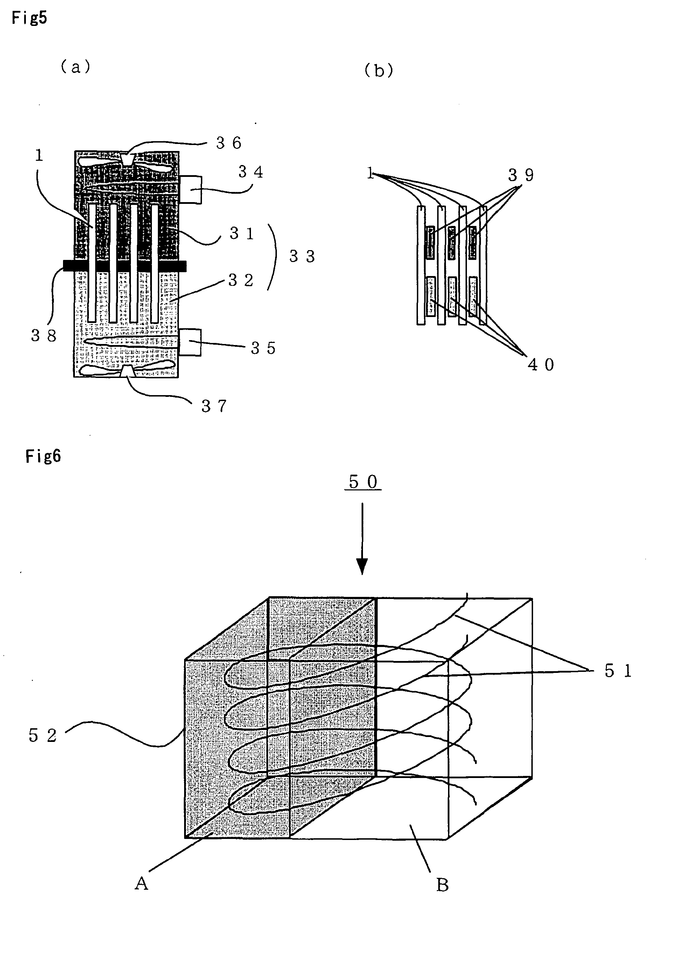

In the nucleic acid amplifier of the present invention, the denaturation-temperature region and the regeneration-temperature region of the base plate can be formed, for example as shown in Fig. 5 (a), by installing a base plate 1 into a temperature-controlling device 33 having a structure in which a thermostatic chamber 31 having a temperature-control means for denaturation 34 and a thermostatic chamber 32 having a temperature-control means for regeneration 35 are partitioned by a partition plate 38. Furthermore, the thermostatic chambers are each provided with a stirrer 36 or 37 in order to stir media in the thermostatic chambers and to keep the temperature uniform.

In addition, as shown in Fig. 5(b), two or more base plates 1 are laminated. Then, the temperature-control means for denaturation 39 and the temperature-control means for regeneration 40 may be arranged between the adjacent base plates or between the base plates every several plates to form the denaturation-temperature region and regeneration-temperature region of the base plate.

Here, the temperature-control means for denaturation and the temperature-control means for regeneration may be kept at predetermined temperatures by means of any temperature-controlling device. Concretely, the temperature-control means may be a thermoelectric device, a thermostat, an electrically heated wire, lamp heater, or the like. In addition, both the temperature-control means for denaturation and the temperature-control device for regeneration may be arranged without contacting with the base plate.

Fig. 6 illustrates still another embodiment of the nucleic acid amplifier of the present invention. The nucleic acid amplifier 50 uses two capillaries 51 as flow channels. The capillaries 51 are placed in the temperature-controlling device 52 having the denaturation-temperature region A and the regeneration-temperature region B such that the capillaries 51 spiral so as to pass alternately through the denaturation-temperature region and the regeneration-temperature region.

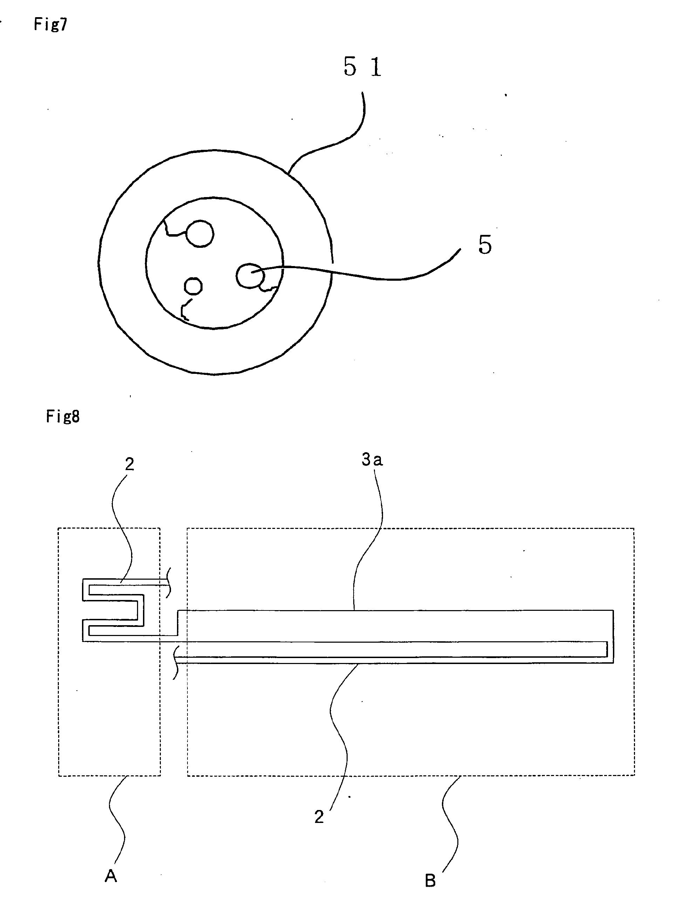

On the inner wall surfaces of the capillaries 51, as shown in Fig. 7, nucleic acid synthetases 5 are directly immobilized.

The capillaries may be preferably made of, but not specifically limited to, a material having comparatively high heat conductivity, stability in the temperature range required for PCR, resistance to erosion with an electrolytic solution or an organic solvent, and hardly adsorbing nucleic acid or protein. For example, glass and plastics can be exemplified. Furthermore, it is preferable that the surface of any of those materials (the surface to be in contact with the reaction solution) be coated with a material, such as polyethylene and polypropylene, which is generally known to hardly adsorbing nucleic acid or protein. Alternatively, it is preferable to prevent the adsorption of nucleic acid or protein by introduction of any molecule rich in hydrophilic functional groups, such as polyethylene glycol (PEG), via a covalent bond or the like.

In addition, any capillary made of a material having the property of semi-permeability that permeates only a low molecular weight substance without passing a high polymer molecule. In this case, a medium of the thermostatic chamber on which such a capillary is mounted may be a solution containing a substrate of a low molecular weight (e.g., dNTP or NTP) to supply a reaction substrate continuously into the capillary. The semi-permeable capillary may be preferably exemplified by hollow fiber available from Mitsubishi Rayon Co., Ltd., Toray Industries. Inc., or the like.

In the present invention, the capillary has an outer diameter of 100 to 1,000 µm, preferably 200 to 500 µm, and an inner diameter of 20 to 600 µm, preferably 50 to 150 µm.

The immobilization of the nucleic acid synthetase on the inner wall of the capillary can be performed by the same method as that of immobilizing the nucleic acid synthetase described above. The nucleic acid synthetase may be immobilized on the entire inner wall surface of the capillary. When the nucleic acid synthetase is immobilized on the whole inner wall surface of the capillary, in general, the nucleic acid synthetase immobilized on the denaturation region may be deactivated by heating or the like, so that it cannot affect the synthetic reaction of nucleic acid. Therefore, there is no problem from a practical standpoint as long as the nucleic acid synthetase immobilized on the regeneration region has activity.

According to this capillary configuration, efforts of loading beads and immobilizing the nucleic acid synthetase only on a specific portion can be saved and the production may be also facilitated.

### Examples

Hereinafter, the present invention will be described concretely with reference to examples, but these examples do not restrict the scope of the present invention.

### <Example 1>

### (Preparation of nucleic acid amplifier)

As illustrated in Fig. 1 and Fig. 2, for obtaining a structure in which a plurality of denaturation regions and regeneration regions were formed alternately in a flow channel, the base plate with flow-channel in which one region of a base plate was defined as a denaturation-temperature region; another region thereof was defined as a regeneration-temperature region; and the flow channel was formed so as to snake its way on the surface thereof and pass through those regions alternately was formed as follows.

That is, polyethylene was subjected to injection molding to form a thin base plate of 1 mm in thickness (35 mm in vertical direction and 70 mm in lateral direction). Then, a groove having no interruption in the length direction and having a width and a depth shown in Table 1 was formed in the surface of a base plate by cutting work to provide the base plate with flow-channel. At this time, a flow channel portion occupied by two adjacent regions, one denaturation region and one regeneration region, was defined as one unit.

**[Table 1]**

| | Width(µm) | Depth(µm) | Length(mm) |
|---|---|---|---|
| Portion of denaturation region in one flow-channel unit | 200 | 200 | 12 |
| Portion of regeneration region in one flow-channel unit (except of beads-filling part) | 200 | 200 | 25 |
| Portion of beads-filling part in one flow-channel unit | 1000 | 200 | 25 |

Fig. 8 is a schematic diagram that represents a groove corresponding to one flow-channel unit. Here, a groove provided as a denaturation region of the denaturation-temperature region A of the base plate has a length of 12 mm along the flow channel. In addition, a groove provided as a regeneration region of the regeneration-temperature region B of the base plate has a length of 50 mm along the flow channel. In addition, a groove in an enlarged diameter portion 3a of the flow channel to be filled with an immobilized nucleic-acid synthesizing enzyme has a width of 1,000 µm, and other part of the groove has a width of 200 µm. A groove without interruption in the length direction, which was formed by cutting work on the polyethylene base plate, is formed such that the grooves corresponding to one flow-channel unit are constructed in a series of 40 units.

On the other hand, an immobilized nucleic-acid synthesizing enzyme to be filled in the enlarged diameter portion 3a of the base plate with flow-channel was prepared as follows.

That is, 1g of a chitosan-beads carrier (trade name "Chitopearl BCW-3001", manufactured by Fuji Spinning Co., Ltd.)(wet weight) having an average particle size of 100 µm was equilibrated in 5 ml of a PBS buffer (137 mM NaCl, 8.1 mM Na₂HPO₄, 2.68 mM KCL, 1.47 mM KH₂PO₄, pH 7.2) at 4°C for 8 hours. The PBS buffer was removed using filtration and then added with 2 ml of a 2.5% aqueous glutaric aldehyde solution for activation at 4°C for 2 hours. After that, the 2.5% aqueous glutaric aldehyde solution was filtered out and the beads were then washed three times with 5 ml of the PBS buffer. Subsequently, after the PBS buffer had been filtered out, 1 ml of 0.1 µg/µl DNA polymerase Klenow fragment (manufactured by Takara Bio Inc.) / PBS buffer was added as an enzyme solution to the beads and then the whole was reacted at 4 °C for 2 hours for immobilization. The enzyme solution was filtered out and washed three times with 5 ml of the PBS buffer, followed by preparing 50% slurry with the PBS buffer.

The immobilized nucleic-acid synthesizing enzyme prepared as described above was dropped to fill a beads-filling part of the base plate with flow-channel at an amount of 2. 5 µl per flow-channel unit using a micropipette. In this case, the immobilized nucleic-acid synthesizing enzyme was considered to occupy approximately half of the capacity in volume of the beads-filling part.

The surface of the base plate with flow-channel, opposite to one on which the groove was formed, was provided with Peltier elements as temperature-control means. That is, a Peltier element required for providing a predetermined area of the base plate as a denaturation-temperature region at 94°C and a Peltier element required for making a predetermined area of the base plate as a regeneration-temperature region at 37°C were mounted above the surface of the base plate with flow-channel.

In addition, for making a flow channel by getting a lid on the base plate with flow-channel, another polyethylene base plate was laminated and then the whole was clipped, thereby providing a base plate for nucleic acid amplification reaction.

Furthermore, a tube for sending solution from a pump that is applicable to high performance liquid chromatography, the pump being provided as a solution-sending device, was connected to a connector attached on the entrance portion of the base plate with flow channel for nucleic acid amplification reaction, thereby providing the nucleic acid amplifier.

### <Example 2>

### (Nucleic acid amplification reaction)

Using the nucleic acid amplifier formed in Example 1, a PCR reaction was carried out. At this time, an aqueous solution containing the following contents was used as a reaction solution.

| Reaction solution: | |
|---|---|
| Template double-stranded DNA (73 bp) | 10 pM |
| Forward primer DNA (18 bp) | 1 µM |
| Reverse primer DNA (18 bp) | 1 µM |
| dATP, dGTP, dCTP, dTTP | each 5 µM |
| MgSO₄ | 10 mM |
| Dithiothreitol | 0.1 mM |
| Tris-HCl (pH 7.2 at 25°C) | 50 mM |

DNA sequence:
Plus chain of template double-stranded DNA: SEQ ID NO: 1
Minus strand of template double-stranded DNA: SEQ ID NO: 2
Forward primer DNA: SEQ ID NO: 3
Reverse primer DNA: SEQ ID NO: 4

Furthermore, out of the reaction solutions, a solution containing no template double-stranded DNA (73 bp) was used as a control.

The reaction solution or the control solution was pre-heated at 94°C for 2 minutes to carry out denaturation and then cooled down to 37°C, followed by being sent into the flow channel of the nucleic acid amplifier of Example 1 at a flow rate of 1 µl/min using a solution-sending device. Here, in consideration of the immobilized nucleic-acid synthesizing enzyme occupying in part of the regeneration region, the ratio in volume between the regeneration region and the denaturation region contained in one unit of the flow channel that is provided on the base plate with flow-channel of the nucleic acid amplifier of Example 1 is approximately 7 : 1. Therefore, the reaction solution or the control solution having passed through the flow channel including 40 units is considered to be subjected to 40-times repetition of the PCR cycle, the cycle consisting of: a denaturation reaction at 94°C for 30 sec; and annealing/extension reaction at 37°C for 3 minutes and 30 seconds.

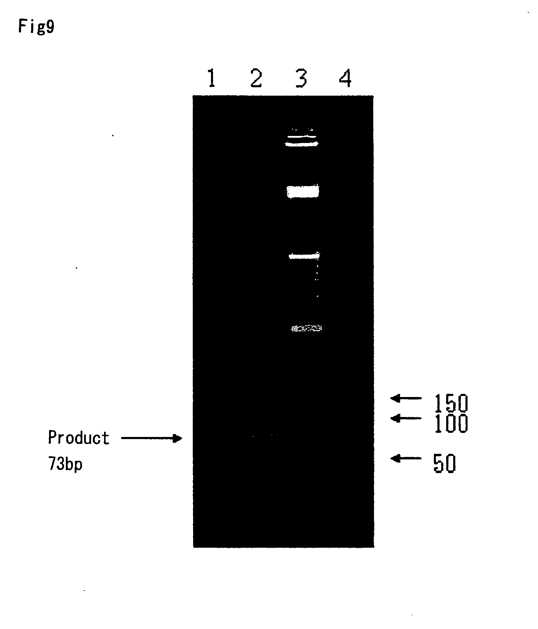

An aliquot of the reaction solution or the control solution, which had passed through the flow channel corresponding to 40 units, and a reaction solution and nucleic acid molecular weight markers before the introduction into the flow channel of the nucleic acid amplifier were subjected to electrophoresis in 3% agarose gel (TAE buffer: 40 mM Tris, 19 mM acetic acid, and 1 mM EDTA). Then, the resulting gel was stained with an aqueous solution of 0.5 µl/ml of ethidium bromide. Fig. 9 shows a photographic image when irradiation with UV (302 nm). In the figure, reference numeral 1 denotes a lane on which a reaction solution before the introduction into the flow channel of the nucleic acid amplifier was electrophoresed, reference numeral 2 denotes a lane on which a reaction solution having passed through the flow channels corresponding to 40 units was electrophoresed, reference numeral 3 denotes a lane on which a nucleic acid molecular weight marker (50 bp ladder) was electrophoresed, and reference numeral 4 denotes a lane on which a control solution having passed through flow channels corresponding to 40 units was electrophoresed.

As is evident from Fig. 9, the double-stranded DNA (73 bp) could not be detected because of its trace amount in the reaction solution before the introduction thereof into the flow channel of the nucleic acid amplifier (lane 1). In addition, DNA was not found in the control solution having passed through the flow channels corresponding to 40 units (lane 4). On the other hand, from the reaction solution having passed through the flow channels corresponding to 40 units, DNA was detected at a position corresponding to 70 to 75 bp with reference to the mobility of the nucleic acid molecular weight marker (lane 3), thereby confirming the amplification of double-stranded DNA (73 bp) in the reaction solution (lane 2).

### [Free text of sequence listing]

SEQ ID NO. 1: Plus chain of template double-stranded DNA having 73 base long to be provided as a template of PCR reaction.
SEQ ID NO. 2: Minus chain of template double-stranded DNA having 73 base long to be provided as a template of PCR reaction.
SEQ ID NO. 3: Forward primer DNA used in PCR for amplification of template DNA.
SEQ ID NO. 4: Reverse primer DNA used in PCR for amplification of template DNA.

### Industrial Applicability

The present invention is applicable to efficient replication and amplification of template nucleic acid.

## Claims

1. A nucleic acid amplifier comprising at least one flow channel therein, in which a reaction solution containing at least a nucleic acid to be used as a template, a nucleic acid to be used as a primer, a phosphate compound, and a metal ion is caused to flow through the flow channel to thereby perform the nucleic acid amplification in the flow channel, **characterized in that** the flow channel comprises:
a denaturation region in which a denaturation reaction is carried out, the denaturation reaction including melting an intramolecularly and/or intermolecularly formed double strand of the nucleic acid to be used as the template;
a regeneration region in which a double strand is formed with the nucleic acid to be used as the template after the double strand thereof is melted and the nucleic acid to be used as the primer; and
a nucleic acid synthetase immobilized in the regeneration region.

2. A nucleic acid amplifier according to claim 1, wherein a means for controlling temperature included by the nucleic acid amplifier is capable of heating the denaturation region and of keeping a temperature of the regeneration region lower than a temperature of the denaturation region.

3. A nucleic acid amplifier according to claim 1 or 2, wherein the nucleic acid synthetase is immobilized on beads, the beads fill at least the regeneration region.

4. A nucleic acid amplifier according to claim 1 or 2, wherein the nucleic acid synthetase is immobilized at least on an inner wall surface of the regeneration region.

5. A nucleic acid amplifier according to any one of claims 1 to 4, wherein the flow channel provides the denaturation region and the regeneration region alternately.

6. A nucleic acid amplifier according to any one of claims 1 to 5, wherein the nucleic acid synthetase has an optimum temperature of 30 to 40°C.

7. A nucleic acid amplifier according to any one of claims 1 to 6, wherein the flow channel provides a circulation flow channel, the circulation flow channel including the regeneration region and the denaturation region.

8. A nucleic acid amplifier according to any one of claims 1 to 7, further comprising a solution-sending device for directionally regulating a flow of the reaction solution, wherein the solution-sending device is controllable to periodically reverse the direction of flow of the reaction solution.

9. A method of amplifying a nucleic acid, the nucleic acid being used as a template in a reaction solution containing at least the nucleic acid to be used as the template, a nucleic acid to be used as a primer, a phosphate compound, and a metal ion, comprising the steps of:
(a) denaturing the nucleic acid to be used as the template by melting anintramolecularly and/orintermolecularlyformed double strand thereof at a predetermined region;
(b) regenerating a double strand by forming the double strand between the nucleic acid obtained in step (a) that to be used as the template wherein the double strand is melted and the nucleic acid to be used as the primer at a region different from the region of the step (a); and
(c) contacting the reaction solution during and/or just after the step (b) with a nucleic acid synthetase immobilized and retained in an active state at a region including the region on which the step (b) is performed.


SEQUENCE LISTING

<110> TAIYO YUDEN CO., LTD

<120> Nucleic Acid Amplification Apparatus and Method

<130> US04-0004

<160> 4

<170> PatentIn version 3.1

<210> 1
<211> 73
<212> DNA
<213> Artificial DNA template

<220>
<223> DNA plus strand template

<220>
<221> misc_feature
<222> (1)..(18)
<223>

<400> 1
ttgcgttgtc tatgccctca cgtagataca tgcattgtgt agctcgagtt ggcgctctca 60

tacagggcta cta 73

<210> 2
<211> 73
<212> DNA
<213> Artificial DNA template

<220>
<223> DNA minus strand template

<220>
<221> misc_feature
<222> (1)..(73)
<223>

<400> 2
tagtagccct gtatgagagc gccaactcga gctacacaat gcatgtatct acgtgagggc 60

atagacaacg caa 73

<210> 3
<211> 18
<212> DNA
<213> Artificial DNA primer

<220>
<223> DNA forward primer

<220>
<221> misc_feature
<222> (1)..(18)
<223>

<400> 3
ttgcgttgtc tatgccct 18

<210> 4

<211> 18
<212> DNA
<213> Artificial DNA primer

<220>
<223> DNA reverse primer

<220>
<221> misc_feature
<222> (1)..(18)
<223>

<400> 4
tagtagccct gtatgaga 18